# EUROPÄISCHE PATENTSCHRIFT

(11) **EP 2 135 616 B1**
(45) Veröffentlichungstag und Bekanntmachung des Hinweises auf die Patenterteilung: **04.05.2016**
(21) Anmeldenummer: 08158602.6
(22) Anmeldetag: 19.06.2008
(51) Int. Cl.: A61K 36/45, A61P 1/12

(54) **Getrocknete Vacciniumfrüchte zur Beeinflussung von Zuständen des Darmes**
Dried bilberries for influencing intestinal conditions
Fruit d'airelle séché destiné à influencer l'état de l'intestin

(43) Veröffentlichungstag der Anmeldung: 23.12.2009
(73) Patentinhaber: Symrise AG, 37603 Holzminden (DE)
(72) Erfinder: Mavric, Elvira Dr., 37603 Holzminden (DE); Krammer, Gerhard Dr., 37603 Holzminden (DE); Rogler, Gerhard, Prof. Dr., CH-8032 Zürich (DE); Piberger, Heidi, 93197 Zeitlam (DE)
(74) Vertreter: Fabry, Bernd

(56) Entgegenhaltungen:
- WO-A-03/084559
- WO-A-2004/087181
- WO-A-2008/004224
- R. HÄNSEL, K. KELLER, H. RIMPLER, G. SCHNEIDER (HRSG.): "Hagers Handbuch der pharmazeutischen Praxis, Band 6, Drogen P-Z, Monographie Vaccinium" 1994, SPRINGER VERLAG , BERLIN , XP002501635 * Seite 1051 - Seite 1067 *
- BLUMENTHAL M: "Bilberry fruit" 23. April 1987 (1987-04-23), THE COMPLETE GERMAN COMMISSION E MONOGRAPHS: THERAPEUTIC GUIDE TO HERBAL MEDICINES,, PAGE(S) 88 , XP008093364 * das ganze Dokument *
- OSMAN N ET AL: "Probiotics and blueberry attenuate the severity of dextran sulfate sodium (DSS)-induced colitis" DIGESTIVE DISEASES AND SCIENCES 200809 US, Bd. 53, Nr. 9, 15. Februar 2008 (2008-02-15), Seiten 2464-2473, XP002501634 ISSN: 0163-2116
- POPOV S V ET AL: "Preventive effect of a peptic polysaccharide of the common cranberry Vaccinium oxycoccos L. on acetic acid-induced colitis in mice" WORLD JOURNAL OF GASTROENTEROLOGY 20061107 CN, Bd. 12, Nr. 41, 7. November 2006 (2006-11-07), Seiten 6646-6651, XP009107721 ISSN: 1007-9327
- Rudolf Fritz Weiss: "Weiss's Herbal Medicine", 1 January 2001 (2001-01-01), Thieme verlag, Stuttgart ISBN: 3-13-129381-0 pages 101-102,
- JURJUS A R ET AL: "Animal models of inflammatory bowel disease", JOURNAL OF PHARMACOLOGICAL AND TOXICOLOGICAL METHODS, ELSEVIER, NEW YORK, NY, US, vol. 50, no. 2, 1 September 2004 (2004-09-01), pages 81-92, XP004572845, ISSN: 1056-8719, DOI: 10.1016/J.VASCN.2003.12.002
- WIRTZ ET AL: "Mouse models of inflammatory bowel disease", ADVANCED DRUG DELIVERY REVIEWS, ELSEVIER BV, AMSTERDAM, NL, vol. 59, no. 11, 30 September 2007 (2007-09-30), pages 1073-1083, XP022312888, ISSN: 0169-409X, DOI: 10.1016/J.ADDR.2007.07.003

## Beschreibung

Die vorliegende Erfindung betrifft Produkte, die zur Beeinflussung bestimmter Zustände des Darmes geeignet sind, nämlich zur Vorbeugung oder Behandlung chronisch entzündlicher Darmerkrankungen, insbesondere chronischer Colitis (colitis ulcerosa).

Ein besonderer Schwerpunkt der vorliegenden Erfindung betrifft die Vorbeugung bzw. Behandlung chronisch entzündlicher Darmerkrankungen, insbesondere chronischer Colitis (colitis ulcerosa).

Chronische Entzündungen können als Ursache verschiedener Krankheiten und Lebensbedingungen erscheinen. Sie können mit unterschiedlichsten Bedingungen wie Arthritis, einigen Arten von Krebs, Colitis, Diabetes Melitus, koronaren Herzkrankheiten, Übergewicht, Alzheimer, Immundisfunktion assoziiert sein.

Eine Ernährungsweise, die auf viel Zucker und Stärke, sowie Fett und trans-Fettsäuren basiert, hat einen direkten Zusammenhang mit chronischer Inflammation. Oxidation von mehrfach ungesättigten Fetten und Fettsäuren *in vitro* und *in vivo* führt zur Bildung von reaktiven Sauerstoffspezies (Radikalen), sowie zur Bildung von Stickoxid. Diese Verbindungen können die erste Phase eines Entzündungsprozesses hervorrufen und fördern. Die Schädigung der DNA kann eine Folge davon sein.

Es gibt zwei enzymatische Wege der Regulierung der Inflammation. Lipoxygenase-Weg (5-LOX) resultiert in der Produktion von Leukotrienen, welche eine proinflammatorische Wirkung aufweisen. Der zweite Weg ist die Cyclooxygenase (COX-1 und COX-2). Ein hoher Level von COX-2 weist auf eine Inflammation hin. Regulierung der Enzyme LOX-5 und COX-2 kann sich bei der Entwicklung/ Suppression von Inflammation positiv auswirken. Weitere Entzündungsmarker sind Tumornekrosisfaktor (TNF-α), Nuklearfaktor- κB (NF- κB), Interleukin-6 (IL-6), Interleukin-17 (IL-17) und Interleukin-l-β (IL1-β). Die Enzyme, Cytokine und ihre Metaboliten erhöhen Produktion von Prostaglandinen und Leukotrienen, welche als interzelluläre Mediatoren fungieren, und im Zusammenhang mit dem Inflammationsprozess stehen.

Der Gastrointestinaltrakt (GIT) ist auf seiner ganzen Länge, insbesondere auch im Bereich des Darmes, anfällig für Entzündungen, weswegen es sehr wichtig ist, entsprechende Vorgänge zu hemmen und Entzündungen vorzubeugen. Ohne Behandlung können schädliche Prozesse zu Irritationen, akuten und chronischen Entzündungen, bis hin zu Krebs führen.

Die Heidelbeere (*Vaccinium myrtillus)* ist (in frischer Form) in der Naturmedizin für ihre heilende Wirkung bei Diarrhö und Magen-Darm-Erkrankungen bekannt. Sie findet Anwendung in verschiedensten medizinischen Gebieten, z.B. Artherosklerose, Katarakt, Diabetes Mellitus, Diarrhoea oder Retinopatie. Anthocyane gehören zu den wichtigsten polyphenolischen Verbindungen in Beeren. Sie können bis zu 5000 mg/kg Frischgewicht in Beeren enthalten sein. Die in der Natur am häufigsten vorkommenden Aglycone (Anthocyanidine) sind: Pelargonidin, Cyanidin, Delphinidin, Peonidin, Petunidin, Malvidin. Beeren enthalten ca. 15 verschiedene Anthocyane.

Anthocyane werden in besonders hoher Konzentration in Früchten (Beeren) von Pflanzen der Gattung *Vaccinium* angetroffen. Einen besonders hohen Anthocyangehalt weisen die Früchte der Heidelbeere (*Vaccinium myrtillus*) auf. Auch die anderen Arten der Gattung *Vaccinium* weisen hohe Gehalte an Anthocyanen auf. Hierzu gehören die amerikanische Heidelbeere oder Kulturheidelbeere (*Vaccinium corymbosum*)*,* Preiselbeere (*Vaccinium vitis-idaea*)*,* gewöhnliche Moosbeere (*Vaccinium oxycoccos*), großfruchtige Moosbeere oder Kranbeere (cranberry) (*Vaccinium macrocarpon*)*,* kleinfrüchtige Moosbeere (*Vaccinium microcarpum*)*,* Alpen Rauschbeere (*Vaccinium gaultheroides*)*,* Rauschbeere (*Vaccinium uliginosum*), Big Huckleberry (*Vaccinium membranaceum*)*,* Red Huckleberry (*Vaccinium parvifolium*), Sparkleberry (*Vaccinium arboreum*), Ohelo-Beere (*Vaccinium reticulatum*), und die kanadische Blaubeere (*Vaccinium myrtilloides*)*.*

Anthocyane sind Verbindungen mit hohem antioxidativen Potential, und wirken als Promotoren der Kollagensynthese, hemmen den Abbau von Kollagen, und stabilisieren das Bindegewebe. Anthocyane sollen bei Wundheilung, Entzündungen der Gefäße und Kollagenabbau wirksam sein (K. J. Kemper, Longwood Herbal Task Force: http://longwoodherbal.org/bilberry/bilberry.Pdf, 1999, Bilberry *Vaccinium Myrtillus*)*.* Aus der Volksmedizin ist es bekannt, dass Holunder- und Moosbeerenextrakte (Cranberries) zur Linderung von Harnwegserkrankungen eingesetzt werden (M., A., Lila, Journal of Biomedicine and Biotechnology, 2004, 5, 306 - 313: Anthocyanins and Human Health: An In Vitro Investigative Approach). Diese Wirkung wird ebenfalls den Anthocyanen zugeschrieben.

Anthocyanreiche Lebensmittel zeigten laut Literatur *in vivo* und *in vitro* eine vorbeugende Wirkung auf verschiedene Krebsarten im GIT (Gastrointestinaltrakt) (oral, esophageal, intestinal, colorectal) (zusammengefasst in J. Agric. Food Chem, 2005, 53, 2859-2866*: Analysis of Anthocyanins in Rat lntestinal Contents- Impact of Anthocyanin Chemical Structure on Fecal Excretion).*

Chronisch entzündliche Erkrankungen der Schleimhaut des Verdauungstraktes stellen ein erhebliches gesundheitspolitisches Problem dar. Es erkranken gerade jüngere Menschen, die dadurch in ihrer gesamten Lebensführung stark beeinträchtigt sind und Zeit ihres Lebens medizinische Versorgung in Anspruch nehmen müssen. Die Ätiopathogenese der chronisch entzündlichen Erkrankungen des Verdauungstraktes ist nicht vollkommen klar. Es wird jedoch als Ursache für die Entstehung eine Störung der Darmbarriere angenommen.

Colitis Ulcerosa und Morbus Crohn sind Entzündungen des Darmes, welche charakteristische Begleiterscheinungen wie Diahrrö, Blutungen im Stuhl, abdominale Schmerzen und Krämpfe, Gewichtsabnahme aufweisen. Die Darmschleimhaut sieht dabei rot und geschwollen aus, blutet schon bei geringster Berührung.

Die oberflächlichste Zellschicht stellen die Epithelzellen der Mukosa dar. Die intestinalen Epithelzellen bilden die größte Kontaktfläche des Körpers zur Außenwelt. Sie resorbieren Nahrung und verhindern gleichzeitig das Eindringen von pathogenen Keimen. Letzteres wird durch eine chronische physiologische Entzündung unterstützt. Diese unterliegt einer Reihe von Kontroll- und Regelmechanismen, um einerseits das Eindringen von pathogenen Keimen und andererseits Schäden durch die Entzündungsmediatoren selbst zu vermeiden. Dazu interagieren die Epithelzellen mit den Zellen des Mukosa-assoziierten Immunsystems.

Intestinale Epithelzellen spielen möglicherweise bei der Pathogenese chronisch entzündlicher Darmerkrankungen eine wichtige Rolle. Das wichtigste Modell der Entstehung chronisch entzündlicher Darmerkrankungen beschreibt folgendes Szenario: Ein Defekt der strukturellen Integrität des intestinalen Epithels führt zu einer Invasion von Antigenen aus dem Darmlumen. Dieser Vorgang kann durch Aktivierung des Mukosa-assoziierten lymphatischen Gewebes in genetisch empfänglichen Patienten eine chronische Entzündung auslösen. Hierfür existieren Belege. Eine Störung des Zell-Zell-Kontakts durch genetische Veränderung des N-Cadherins oder des Keratin 8 löst eine chronische Darmentzündung aus. Epithelzellen besitzen eine Vielzahl von Rezeptoren zur Signalaufnahme. Hierzu zählen vor allem Rezeptoren zur Erkennung bakterieller Motive, so genannte Pattern Recognition Rezeptoren.

Ein solcher Erkennungsrezeptor für bakterielle Motive ist das so genannte NOD2/CARD15 Protein. NOD2/CARD15 ist ein Mitglied der NBS-LRR-Protein-Familie (für nucleotide-binding site and leucine-rich repeat), deren Mitglieder alle eine Rolle bei der intrazellulären Erkennung von Mikroben und ihren Bestandteilen spielen und zu denen z. B. auch Apaf-1 und CARD4/NOD1 zählen, die möglicherweise ebenso bei bestimmten Patienten eine Rolle spielen können. Wenn bakterielle Bestandteile an NOD2/CARD15 binden, führt das normalerweise zu einer Aktivierung des proentzündlichen Transkriptionsfaktors NF-kappaB.

In Ulcerationen bei Patienten mit M. Crohn wurden adhärente E. coli-Stämme gefunden. Generell sind bei Patienten mit chronisch entzündlichen Darmerkrankungen (CED; inflammatory bowel disease, IBD) wesentlich mehr Bakterien den intestinalen Epithelzellen unmittelbar anliegend als in der normalen Mukosa, die von einer Mukus-Schicht vor dem Kontakt mit Bakterien geschützt ist. Auch diese Beobachtung unterstützt die Hypothese von der Bedeutung der bakteriellen Translokation in der Pathogenese der CED.

Die heute verfügbaren Therapien zur Behandlung von Morbus Crohn und Colitis Ulcerosa können die Krankheitssymptome lindern, aber nicht heilen. Die meisten Therapien enden in einer Resistenz dem Antibiotikum gegenüber und dem operativen Eingriff.

JP 2007/077122 beschreibt Anwendung von Pflanzen- Proanthocyanidinen wie beispielsweise Apfel, Birne, Aprikose, Traube, Guave, Hopfen, Gerste oder Adzuki Bohne für die Prävention von Darmentzündungen, speziell im Fall von Colitis ulcerosa. Die empfohlene tägliche Aufnahme beträgt 100 bis 2500 mg Apfelextrakt oder Apfel-Proanthocyanidinen. Die Wirkung der Apfel Proanthocyanidine wurde an Mäusen mit einer durch Dextransulfat (DSS, 2,5 %) verursachten akuten Colitis Ulcerosa in einem Zeitraum von 20 Tagen bestätigt.

Modelle der DSS-induzierten Colitis (akut oder chronisch) sind schnell, einfach durchführbar, gut reproduzierbar und preiswert. Sie erlauben die dynamische Studie des Entzündungsprozesses von der Entstehung bis zur Remission, und sind somit für Untersuchungen der epithelialen Regeneration und Wundheilung sowie zum Arzeneimittelscreening gut geeignet.

WO 2007/130893 beschreibt die Behandlung von entzündlichen Darmkrankheiten, (inklusive Morbus Crohn und Ulcerative Colitis) mit Hilfe von polymeren Proanthocyanidinen aus Croton und Calophyllum Spezies. Das Proanthocyanidin Crofelmer (CAS 148465-45-6) inhibiert selektiv COX-2 und COX-1 Enzyme.

WO 01/015553 beschreibt die Anwendung von Pflanzen der Gattung *Vaccinium-* bei Entzündungen (anti-inflammatorische Wirkung), Schmerzreduktion und Hemmung von COX-2. Angewendet werden Extrakte der Vaccininium-Pflanzen, die reich an Flavonoiden und Anthocyanen sind. Des Weiteren wird dort angegeben, dass die verwendeten Pflanzenextrakte eine antiinflammatorische Aktivität aufweisen. Die antiinflammatorische Wirkung basiert auf Cyanidin-3-glucosid, Cyanidin-3-glcosylrutinosid, Cyanidin-3-gentibiosid, Cyanidin-3-rutinosid, Cyanidin-3-sophorosid, Peonidin-3-rutinosid, Peonidin, Cyanidin, Pelargonidin, Delphinidin, Petunidin, Malvidin, Kaempferol, Hesperidin, Gentiodelphin, Platyconin, Cinerarin und deren Mischungen.

WO 03/084559 offenbart die Verwendung getrockneter Vacciniumpflanzenteile.

WO 2006/083666 beschreibt die Anwendung einer speziellen Mischung aus 38,0 % Grünteeextrakt, 24,0 % Traubenkernextrakt, 21,9 % Heidelbeerextrakt, 11,4 % Apfelextrakt und 4,7 % Curcumin für die Behandlung von entzündlichen Prozessen im GIT(Magen-Darm-Trakt). Die Mischung beinhaltet aktive Komponenten, unter anderem Curcuminoide, Proanthocyanidine, Quercetin und Catechine. Es wird offenbart, dass eine spezifische Mischung aus Curcumin, Grünteeextrakt, Traubenkernextrakt, γ-Tocopherolen und Blaubeer- und/ oder Heidelbeerextrakt, die entzündlichen Vorgänge im oberen GIT reduziert. Die Effektivität der Mischung kann dabei durch Zugabe von Triglyceriden, kurzkettigen und langkettigen Fettsäuren, sowie Omega-3 Fettsäuren gesteigert werden. Die Untersuchungen beschränken sich auf die Regulierung der Entzündungsmarker.

Eine weitere Studie beschreibt die Wirkung von Heidelbeer- Anthocyanosiden mit 25 % Anthocyanidingehalt auf unterschiedlich hervorgerufene Magengeschwüre im Tierversuch. Die Anwendung von Heidelbeer-Extrakt wird bei der Behandlung von akuten und chronischen Magen-Ulcera empfohlen (A. Christoni, M., J., Magistretti, 1987, II Farmaco, Anno XLII, Nr. 2, 29 - 43, Antiulcer and Healing Activity of *Vaccinium Myrtilus* Anthocyanosides; M., J., Magistretti, M., Conti, A., Christoni, 1988, Arzneim.-Forsch./ Drug Res., 38 (I), Nr. 5, 686 - 690, Antiulcer Activity of an Anthocyanidin from *Vaccinium Myrtilus;* K. J. Kemper, Longwood Herbal Task Force: http://longwoodherbal.org/bilberry/bilberry.pdf, 1999, Bilberry *Vaccinium Myrtillus*).

"Hagers Handbuch der pharmazeutischen Praxis, Band 6, Drogen P-Z, Monographie Vaccinium", 1994, Springer Verlag, offenbart die Verwendung von reifen, getrockneten Heidelbeerfrüchten der Stammpflanze Vaccinium myrtillus L. zur Unterstützung der Therapie von und bei spezifischen akuten Durchfallerkrankungen bei Schulkindern und Erwachsenen.

"Bilberry Food" (23. April 1987, The complete German commission e monographes: therapeutic guide to herbal medicines) offenbart die Verwendung von "Bilberry Food" (Heidelbeeren), den getrockneten, reifen Früchten von Vaccinium myrtillus L., bei nicht spezifischer, akuter Diarrhoe.

"Probiotics and blueberry attenuate the severity fo dextran sulfate sodium (DDS)-induced colitis", Digestive diseases and sciences, 200809 US, Bd. 53, Nr. 9, Seiten 2464-2473, offenbart die Verwendung von gefriergetrocknetem Blaubeerpulver, das aus ausgepresstem Saft Blaubeeren gewonnen worden ist, zur Behandlung allein oder in Kombination mit bestimmten Bakterienstämmen von in Ratten erzeugter akuter Colitis.

"Preventive effect of a peptic polysaccharide of the common cranberry Vaccinium oxycoccos L. on acetic acid-induced colitis in mice", World Journal of gastroenterology, Bd. 12, Nr. 41, 7, 2006, Seiten 6646-6651, offenbart einen präventiven Effekt einer Polysacharid-Fraktion aus Vaccinium oxycoccos gegenüber Colitis ulcerosa. Die Polysacharid-Fraktion wird aus reifen Beeren von Vaccinium oxycoccos extrahiert, wobei die extrahierte Polysacharid-Fraktion einen präventiven Effekt bei induzierten Colitis ulcerosa in Mäusen zeigt. Die Colitis ulcerosa wurde in den Versuchstieren druch rektale Injektion von 5%-iger Essigsäure hervorgerufen und 24 Stunden später die Darmschädigung analysiert.

WO 2008/004224 betrifft Verfahren und synergistische Zusammensetzungen, die eine Nitroimidazol-Antibiotikum und ein Makrolidantibiotikum, gegebenenfalls in Kombination mit Polyphenolverbindungen aus Pflanzen oder Früchten enthalten, zur Behandlung von chronischen entzündlichen Erkrankungen des Gastrointestinaltrakts wie Morbus Crohn.

WO 2004/087181A betrifft eine Wirkstoffkombination, umfassend mindestens ein ω3-fettsäurehaltiges Öi und mindestens einen polyphenolhaltigen Pflanzenextrakt, wobei diese gleichzeitig einen Mangel an ω3-Fettsäuren und an antioxidativen Polyphenolen ausgleicht, einem solchen Mangel vorbeugt oder einen erhöhten Bedarf dieser Substanzen bei entzündlichen und/oder immunologischen sowie metäbolischen Erkrankungen und Tumorleiden abdeckt. Diese Wirkstoffkombination wirkt durch die Hemmung der Produktion von proinflammatorischen Eicosanoiden und der Neutralisation von freien Radikalen gleichzeitig zwei wichtigen an der Pathogenese von Entzündungsreaktionen und Stoffwechselerkrankungen beteiligten Faktoren entgegen. Ausserdem hat sie den Vorteil, dass die oxidationsempfindlichen ω3-Fettsäuren durch die antioxidative Wirkung der Polyphenole und/oder Vitamine sowohl bei der Lagerung als auch nach der Inkorporation in Zellmembranen vor einer Degradation geschützt werden.

"Weiss's Herbal Medicine", 2001, Seiten 101-102, Thieme Verlag beschriebt die Verwendung von getrockneten Heildelbeeren zur Behandlung akuter Diarrhöen.

Die Dokumente Journal of Pharmacological and Toxicological Methods, Band 50, Nr. 2, 2004, Seiten 81-92, und Advanced Drug Delivery Reviews, Bd. 59, Nr. 11, 2007, Seiten 1073-1083 geben ein Übersicht der letzten Fortschritte bezüglich der Tiermodellen zum Studium von chronisch-entzündlichen Darmerkrankungen.

Im Stand der Technik beschriebene Behandlungsmethoden für chronisch entzündliche Darmerkrankungen (Morbus Crohn; Colitis ulcerosa) basieren auf der Verabreichung pharmazeutischer Produkte wie z.B. 5-Acetylsalicysäure (5-ASA) (Wirkstoff Mesalazin) und ihrer Abkömmlinge über mehrere Monate bzw. auf einer Kombination aus Medikamenten und unterschiedlichen polyphenolhaltigen Pflanzen oder verschiedenen Pflanzenmischungen bzw. Extrakten, die einen hohen Anteil an Polyphenolen, Anthocyanen und/ oder Anthocyanidinen aufweisen.

5-Acetylsalicysäure wird bei milder bis mittel schwerer Colitis ulcerosa eingesetzt. Die Dosierung erfolgt mit 6 g/d. Bei schwererer Erkrankung kann 5-ASA auch in Kombination mit Steroiden benutzt werden. Als Nebenwirkungen können Durchfälle, Blutbildveränderungen, allergische Reaktionen, Übelkeit, Erbrechen, Durchfall oder Juckreiz auftreten.

Die chronische Colitis muss von der akuten Colitis unterschieden werden. Wie sich jetzt in eigenen Untersuchungen bestätigt hat, sind Informationen aus dem Stand der Technik, welche die Behandlung einer akuten Colitis betreffen, gar nicht oder allenfalls nur sehr begrenzt übertragbar auf die Behandlung chronischer Colitis, und umgekehrt. Die vorliegende Erfindung betrifft insbesondere die Behandlung chronischer Colitis und die Beeinflussung von Zuständen des Darmes, die mit der chronischen Colitis (colitis ulcerosa) in engem Zusammenhang stehen. Die Behandlung der akuten Colitis ist nicht Gegenstand der vorliegenden Erfindung; soweit im Folgenden auf Untersuchungen zur Behandlung akuter Colitis eingegangen wird, handelt es sich um vergleichende Untersuchungen.

Repräsentativ für chronisch entzündliche Darmerkrankungen ist das Modell der chronischen DSS-Colitis. Im Rahmen der vorliegenden Erfindung wurde ein Modell der chronischen DSS-Colitis eingesetzt, bei dem DSS (Dextransulfat Natrium) Mäusen viermal über jeweils eine Woche mit jeweils einer Woche Pause verabreicht wurde. Vier Wochen nach einer letzten DSS-Gabe, also insgesamt nach zehn bis zwölf Wochen, erfolgte die therapeutische Behandlung der Mäuse. Zu diesem Zeitpunkt besteht eine chronische Entzündung, die sich deutlich von einer akuten Schädigung unterscheidet. So führt in einem akuten DSS-Colitis-Modell der Maus eine Neutralisierung von TNF mit Anti-TNF-Antikörpern zu einer erhöhten Darmschädigung, zu einer Verschlechterung der Barrierefunktion und zu einer erhöhten Letalität. Hingegen reduziert die Neutralisierung von TNF im chronischen DSS-Colitis-Modell der Maus die Darmschädigung, verbessert die Darmbarriere und erhöht die Überlebensdauer.

Es war die Aufgabe der vorliegenden Erfindung, Produkte zur Beeinflussung von Zuständen des Darmes anzugeben, insbesondere sollte ein Produkt zur Vorbeugung oder Behandlung chronisch entzündlicher Darmerkrankungen angegeben werden und bevorzugt eines zur Behandlung chronischer Colitis (colitis ulcerosa).

Erfindungsgemäß wurde diese Aufgabe gelöst durch ein Produkt auf Basis von getrockneten Vacciniumfrüchten, wobei das Produkt ausgewählt ist aus der Gruppe bestehend aus:
(i) getrocknete Vacciniumfrüchte, (ii) getrockneter Teil von Vacciniumfrüchten oder (iii) Extrakt davon,
zur Anwendung in der therapeutischen Beeinflussung von Zuständen des Darmes, wobei die Beeinflussung keine Behandlung der akuten Colitis ist und der Vorbeugung oder Behandlung chronisch entzündlicher Darmerkrankungen, insbesondere chronischer Colitis (colitis ulcerosa) dient.

Überraschenderweise hat sich in eigenen Untersuchungen gezeigt, dass getrocknete Vacciniumfrüchte, getrocknete Teile von Vacciniumfrüchten oder Extrakte solcher getrockneten Produkte in nicht vorhersehbarer Weise dazu geeignet sind, colitis ulcerosa vorzubeugen und zu behandeln.

Bei regelmäßigem, insbesondere täglichem, Verzehr eines erfindungsgemäßen Produktes tritt schon innerhalb weniger Tage eine deutliche Verbesserung des Darmzustandes ein.

In eigenen Untersuchungen wurden insbesondere getrocknete Heidelbeere, getrocknete Blaubeere und Extrakte dieser getrockneten Früchte erfolgreich bei der Behandlung einer chronischen Colitis im Tiermodell angewendet. Untersucht wurde zudem der Einfluss dieser Produkte auf die Reparaturmechanismen der geschädigten Darmwand. In eigenen Untersuchungen wurde auf die zusätzliche Verabreichung von Medikamenten verzichtet, so dass der alleinige Einsatz der besagten Produkte auf Basis von getrockneten Vacciniumfrüchten als bioaktiver Komponente für die erzielten Resultate verantwortlich ist.

Die vorliegende Erfindung betrifft neben dem Produkt auf Basis von getrockneten Vacciniumfrüchten zur Beeinflussung von Zuständen des Darmes auch eine entsprechende Verwendung eines Produktes auf Basis von getrockneten Vacciniumfrüchten zur Herstellung eines Medikaments zur Beeinflussung von Zuständen des Darms.

Hinsichtlich der im Rahmen der Erfindung einsetzbaren Produkte auf Basis von getrockneten Vacciniumfrüchten und hinsichtlich der von der Erfindung umfassten Beeinflussungen von Zuständen des Darmes gelten die zu den erfindungsgemäßen Produkten gemachten Aussagen entsprechend. Bevorzugte Ausgestaltungen ergeben sich auch aus den beigefügten Patentansprüchen.

Die nachfolgenden Ausführungen zu bevorzugten erfindungsgemäßen Produkten gelten entsprechend auch für die erfindungsgemäßen Verwendungen von Produkten auf Basis von getrockneten Vacciniumfrüchten.

In einem erfindungsgemäßen Produkt sind die getrockneten Vacciniumfrüchte vorzugsweise ausgewählt aus der Gruppe bestehend aus:
amerikanische Heidelbeere, Kulturheidelbeere *(Vaccinium corymbosum*)*;*
Preiselbeere (*Vaccinium vitis-idaea*)*;*
gewöhnliche Moosbeere (*Vaccinium oxycoccos*);
großfruchtige Moosbeere, Kranbeere (cranberry) (*Vaccinium macrocarpon*);
kleinfrüchtige Moosbeere (*Vaccinium microcarpum*);
Alpen Rauschbeere (*Vaccinium gaultheroides*);
Rauschbeere (*Vaccinium uliginosum*);
Big Huckleberry *(Vaccinium membranaceum*);
Red Huckleberry *(Vaccinium parvifolium*);
Sparkleberry (*Vaccinium arboreum*)*;*
Ohelo-Beere (*Vaccinium reticulatum*)*;*
kanadische Blaubeere *(Vaccinium myrtilloides*)*;*
und deren Mischungen.

Im Rahmen der vorliegenden Erfindung ist der Extrakt der getrockneten Heidelbeere (Bilberry) besonders bevorzugt.

Unter den erfindungsgemäßen Produkten besitzen die (i) getrockneten Vacciniumfrüchte bzw. die (ii) getrockneten Teile von Vacciniumfrüchten vorzugsweise einen Feststoffgehalt von 90 Gew. % oder mehr. Bevorzugt sind ebenfalls Extrakte derartiger getrockneter Vacciniumfrüchte oder derartiger getrockneter Teile von Vacciniumfrüchten.

Insbesondere überraschend war es, dass sich im Rahmen eigener Untersuchungen gezeigt hat, dass sich erfindungsgemäße Produkte (insbesondere bevorzugte erfindungsgemäße Produkte, wie vorstehend definiert) zur Vorbeugung oder Behandlung chronischer DSS-induzierter Colitis hervorragend eignen, jedoch keine hervorragenden Ergebnisse bei der Behandlung akuter DSS-induzierter Colitis erzielt werden.

Die eigenen Untersuchungen umfassten insbesondere eine Tierstudie, mit der die Wirkung getrockneter Heidelbeeren und eines standardisierten Heidelbeerextraktes 95 % (Extrakt getrockneter Heidelbeeren) auf die Entwicklung von akuter und chronischer DSS-induzierter Colitis untersucht wurde. Die Untersuchungen zu den Markern und Parametern der akuten Colitis sind als Vergleich zum Modell der chronischen Colitis durchgeführt worden. In den Tierstudien wurde erstmalig eine erfolgreiche Behandlung von chronischer Colitis mit Hilfe von getrockneten Heidelbeeren erreicht.

Extrakte getrockneter Vacciniumfrüchte besitzen allenfalls eine geringe Fett- bzw. Öllöslichkeit. Zu den wertgebenden Inhaltsstoffen dieser Extrakte zählen die Anthocyane, die einen Anteil von vorzugsweise 0,1-30 Gew.-% des trockenen Extraktes ausmachen. Die in der Gattung Vaccinium vorkommenden Anthocyane Cyanidin, Delphinidin, Malvidin, Petunidin und Peonidin liegen überwiegend glycosidisch gebunden als Cyanidin-3-arabinosid, Cyanidin-3-galactosid, Cyanidin-3-glucosid, Del-phinidin-3-arabinosid, Delphinidin-3-galactosid, Delphinidin-3-glucosid, Malvidin-3-arabinosid, Malvidin-3-galactosid, Malvidin-3-glucosid, Petunidin-3-arabinosid, Petunidin-3- galactosid, Petunidin-3-glucosid, Peonidin-3- arabinosid, Peonidin-3-galactosid, Peonidin-3-glucosid vor. Als weitere Inhaltstoffe des Extraktes sind vor allem Saccharide, organische Säuren, und weitere Polyphenole, wie Flavonoide und Gerbsäuren (Tannine) sowie Vitamine zu nennen.

Extrakte aus getrockneten Vacciniumfrüchten bzw. getrockneten Teilen von Vacciniumfrüchten, die als erfindungsgemäßes Produkt zur Beeinflussung von Zuständen des Darmes eingesetzt werden können, können durch an sich bekannte Extraktionsverfahren aus den getrockneten Früchten (Beeren) der Pflanzen bzw. deren Teilen gewonnen werden. Hierzu zählen z.B. die Mazeration oder Perkolation. Als Extraktionsmedium kann z. B Wasser und Ethanol bzw. Mischungen daraus verwendet werden. Anstelle von Ethanol können auch Methanol und andere wasserlösliche Lösungsmittel verwendet werden. Die Temperaturwahl und mechanische Desintegration der Früchte können die Extraktion unterstützen. Nach dem Stand der Technik empfiehlt sich auch die mechanische Desintegration der getrockneten Früchte z.B. durch Rührer, Homogenisatoren oder Ultraschall. Ferner können weitere extraktionsfördernde Stoffe, wie Säuren, Basen und Enzyme eingesetzt werden.

Der Begriff "getrocknete Heidelbeere" umfasst im Rahmen des vorliegenden Textes die getrocknete amerikanische Heidelbeere oder Kulturheidelbeere (*Vaccinium corymbosum),* die getrocknete kanadische Blaubeere (*Vaccinium myrtilloides*) und die getrocknete eurasische Blaubeere (*Vaccinium myrtillus*)*.* Die Trocknung erfolgte im Rahmen eigener Untersuchungen konventionell bei 20 - 25 °C für 5- 6 Tage. Die getrockneten Heidelbeeren können für entsprechende Applikationen vermahlen und eingesetzt werden.

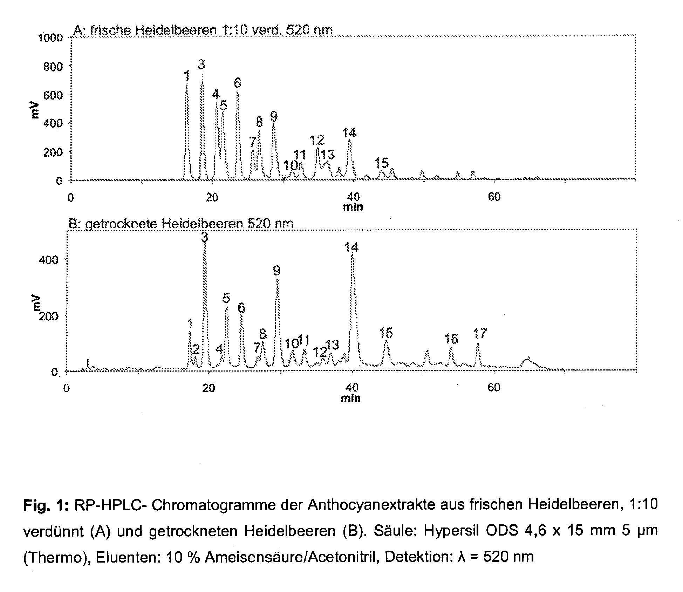

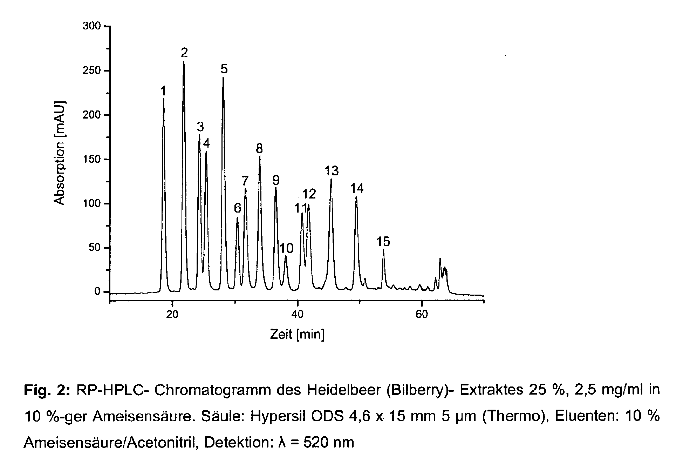

Im Rahmen eigener Untersuchungen zur Gesamtzusammensetzung wurde in getrockneten Heidelbeeren ein Trockenmassegehalt von 91,4 % ermittelt. Protein, Fett, Glucose, Fructose, Asche und Gesamtsäure stellen in ihrer Summe ca. 40 % der Trockenmasse der getrockneten Heidelbeeren dar. Der noch unbekannte Rest ist auf bisher nicht analysierte Bestandteile zurückzuführen. Aufgrund der Zusammensetzung von frischen Heidelbeeren wird jedoch vermutet, dass der Großteil des Restes aus Rohfaser-Komponenten wie Cellulose, Pektin, Pentosanen oder Tanninen besteht. Daneben sind vermutlich geringe Anteile von infolge des Trocknungsprozesses entstehenden Reaktionsprodukten z.B. aus Zuckern und Proteinen oder Polyphenolen vorhanden. In eigenen Untersuchungen wurde die Identifizierung und Quantifizierung der Anthocyane in getrockneten und (zum Vergleich) frischen Heidelbeeren nach der methanolischen Extraktion (5 % Ameisensäure) mittels RP-HPLC-UV/Vis und RP-HPLC-MS/MS vorgenommen. Die Anthocyangehalte wurden mittels externer Kalibrierung unter Verwendung von Standardsubstanzen ermittelt. Die beigefügte Figur 1 zeigt die Anthocyanprofile der Extrakte. Die identifizierten Anthocyane sowie die Anthocyangehalte in den Extrakten aus getrockneten und frischen Heidelbeeren sind in Tabelle 1 zusammengefasst. Die Extrakte aus getrockneten Heidelbeeren stellen erfindungsgemäße Produkte dar, die unmittelbar zur Beeinflussung von Zuständen des Darmes eingesetzt werden können. Die Extrakte aus frischen Heidelbeeren dienen dem Vergleich.

Der Vergleich der Chromatogramme der Extrakte von frischen Heidelbeeren bzw. getrockneten Heidelbeeren (Fig. 1) zeigt, dass sich die wesentlichen Anthocyan-Bestandteile frischer Wildheidelbeeren auch im Chromatogramm des Extraktes aus getrockneten Heidelbeeren wiederfinden. Allerdings sind deutliche Unterschiede in den Peak-Flächen und somit im Gehalt der Anthocyane erkennbar. Im Extrakt aus getrockneten Heidelbeeren wurden zudem Minoranthocyane identifiziert, bei denen es sich um eine Delphinidin-hexose-pentose (Fig. 1, Peak 2) sowie um mehrere Anthocyanpentosen, vermutlich Anthocyanxyloside (Fig. 1, Peak 16, Peak 17)handelt. Vermutlich sind die Unterschiede in der Zusammensetzung der jeweiligen Extrakte dafür verantwortlich, dass die Extrakte getrockneter Heidelbeeren in eigenen Studien (siehe dazu unten) zu besseren Resultaten führten als die Extrakte frischer Heidelbeeren. Diese Erkenntnis ist in hohem Maße überraschend und war angesichts des Standes der Technik nicht vorhersehbar.

Der wesentliche Inhalt der Chromatogramme ist in der beigefügten Tabelle 1 zusammengefasst, welche auch entsprechende Daten zu einem Heidelbeerextrakt 25 % umfasst (standardisierter Extrakt der Kaden Biochemicals GmbH, welcher mindestens 25 Gew.-% Anthocyane enthält).

Die Konzentration der Anthocyane in den getrockneten Heidelbeeren ist um Faktor 7,56 bei Mv-3-gal bis Faktor 217,25 bei Cy-3-gal niedriger als in frischen Heidelbeeren. Verglichen mit den Anthocyanen des Heidelbeerextraktes 25 % sind diese in getrockneten Heidelbeeren fast zu vernachlässigen.

In zwei voneinander unabhängigen Tierstudien (TS1, TS2, siehe unten) wurde mittels DSS (i) eine akute bzw. (ii) eine chronische Colitis hervorgerufen. Nach Induktion der Colitis wurden die Mäuse mit getrockneten Heidelbeeren (erfindungsgemäß) bzw. mit einem Heidelbeerextrakt 25 % (Vergleich) gefüttert. Nach einem Behandlungszeitraum von zwei Wochen in beiden Modellen (akut; chronisch) wurden verschiedene Parameter der Erkrankung untersucht.

Überraschenderweise zeigte sich, dass eine Behandlung von chronischer Colitis mit getrockneten Heidelbeeren einen hervorragenden Effekt bewirkt. Der Effekt der getrockneten Heidelbeeren liegt dabei innerhalb derselben Größenordnung wie der einer TNF-Blockierung oder einer Beigabe von Steroiden. Ein ähnlicher Effekt wurde mit Heidelbeerextrakt 25 % nicht erreicht, es wurde vielmehr lediglich eine leichte Verbesserung der chronischen Colitis bewirkt.

Der Einsatz getrockneter Heidelbeeren bei der Behandlung akuter Colitis erwies sich hingegen als nicht geeignet; hinsichtlich der akuten Colitis wirkte der Einsatz eines Heidelbeerextraktes 25 % deutlich besser, was vermutlich am hohen Gehalt an Anthocyanen liegt, die im Heidelbeerextrakt 25 % enthalten sind und für die in der Literatur ihre anti-inflammatorische und anti-oxidative Wirkung beschrieben ist.

Die vorliegende Erfindung betrifft neben den vorangehend beschriebenen Produkten auf Basis von getrockneten Vacciniumfrüchten, die zur Beeinflussung von Zuständen des Darmes eingesetzt werden, auch Zubereitungen, die ein Produkt auf Basis von getrockneten Vacciniumfrüchten umfassen, wie es hinsichtlich der stofflichen Zusammensetzung vorstehend beschrieben ist. Die vorliegende Erfindung betrifft Produkte, die (i) getrocknete Vacciniumfrüchte, (ii) getrocknete Teile von Vacciniumfrüchten oder (iii) Extrakte davon sind. In den erfindungsgemäßen Zubereitungen sind neben diesen Produkten ein oder mehrere weitere Substanzen enthalten, die zur Beeinflussung von Zuständen des Darmes geeignet sind, wobei die Beeinflussung die Vorbeugung oder Behandlung chronisch entzündlicher Darmerkrankungen ist, insbesondere chronischer Colitis (colitis ulcerosa);
- Substanzen zur Behandlung einer bei Darmerkrankungen auftretenden Mangelerscheinung.

Hierbei ist der Mangel insbesondere ein Mangel an einer oder mehrerer Substanzen, die ausgewählt sind aus der Gruppe bestehend aus: Kalium, Natrium, Eisen, Calcium, Vitamin D und Folsäure.

In erfindungsgemäßen Zubereitungen wird das Produkt auf Basis von getrockneten Vacciniumfrüchten ganz besonders bevorzugt kombiniert mit einer oder mehreren Substanzen ausgewählt aus der Gruppe bestehend aus:
Probiotische Bakterien (z.B. Lactobacillen, Bifidobakterien, Enterokokken);
Präbiotika (z.B. Inulin, Fructooligosaccharide);
Synbiotika (Pro- und Präbiotika);
Ballaststoffe (Cellulose, Stärke, resistente Stärke, Fasern, wie beispielsweise Apfelfasern);
Molkenproteine, Sojaproteine;
Mineralstoffe (insbesondere Ca, Mg, wobei eine Kombination von Ca, Mg und Inulin besonders bevorzugt ist);
Tocopherole (z.B. Vitamin E, Vitamin-E-acteat);
Vanille und Vanille-Extrakte;
Omega-3 - Fettsäuren (vorzugsweise Fischöl) und pflanzliche omega-3-Fettsäuren;
Extrakte vom Apfel, Traubenkernen, Grüntee, Rosmarin, Estragon, Thymian, Meerrettich, und/oder Macis;
Tannine;
Extrakte von Tomate, Melone, und/oder Hagebutte (insbesondere Lycopin-haltige Extrakte);
Aubergine, Rhabarber, rote Zwiebel, Rotkohl, Schwarzkarotte;
beta-Carotin;
sogenannte Superfruits, vorzugsweise Açai, Noni, Goji, Granatapfel, Mangosteen, Johannisbeeren, Erdbeeren, Aronia, Holunderbeeren, bevorzugt in getrockneter Form;
Sojaisoflavone.

Erfindungsgemäße Zubereitungen (insbesondere die vorstehend als bevorzugt bezeichneten Zubereitungen) sind vorzugsweise ausgewählt aus der Gruppe bestehend aus:
fruchtsafthaltige Getränke; gemüsesafthaltige Getränke; Backwaren; Süßwaren; Snacks; Instant-Produkte; Suppen; Saucen; Würzmischungen; Speiseeis; Fruchtzubereitungen; Desserts; Milchprodukte; Sojaprodukte; Cerealien; Nahrungsergänzungsmittel; pharmazeutische Produkte.

Fruchtsafthaltige Getränke sind dabei insbesondere Fruchtsäfte und Smoothies (Vollfruchtgetränke). Gemüsesafthaltige Getränke sind insbesondere Säfte von Rote Beete und Schwarzkarotte. Backwaren sind insbesondere Kuchen, Waffeln und Kekse. Süßwaren sind insbesondere Lutschbonbons, Kaugummis, Fruchtgummis, Kaubonbons, (Erfrischungs-)Dragees, Komprimate, Hartkaramellen, Schokocremes, Konfekt und Schokolade. Instant-Produkte sind insbesondere Instant-Mahlzeiten sowie andere Instant-Produkte, z. B. Getränkepulver und -granulate. Fruchtzubereitungen sind insbesondere Marmelade, Konfitüre, Fruchtsoßen. Desserts sind insbesondere Puddings und Götterspeise. Milchprodukte umfassen insbesondere Quark, Yoghurt, Milchdrinks, Molkezubereitungen. Cerealien sind insbesondere Cornflakes, Müsli und Müsliriegel. Nahrungsergänzungsmittel und pharmazeutische Produkte können insbesondere in Form von Lutschtabletten, Hals- oder Hustenbonbons, pharmazeutischen Pulvern, Tabletten oder Granulaten vorliegen.

Der Untersuchungszeitraum bei der akuten Colitis belief sich auf insgesamt drei Wochen, wogegen der Zeitraum der chronischen Colitis zwölf bis vierzehn Wochen betrug. Die Behandlung beider Colitis-Modelle wurde mit getrockneten Heidelbeeren, und mit einem Vacciniumextrakt 25 % in getrennten Studien vorgenommen.

Vorzugsweise enthält das Normalfutter der Mäuse in beiden Colitis Modellen erfindungsgemäß:
a) 5 - 60,0 Gew.-% getrocknete Heidelbeeren
b) 40,0 - 95 Gew.-% Normalfutter (Trockenfutter)
bzw.
a) 5 - 50,0 Gew.-% Heidelbeerextrakt 25 %
b) 50,0 - 95 Gew.-% Normalfutter (Trockenfutter)

Nach der Induktion der Colitis werden die Mäuse erfindungsgemäß zur Behandlung mit getrockneten Heidelbeeren bzw. mit dem Heidelbeerextrakt 25 % gefüttert. Nach einem Behandlungszeitraum von zwei Wochen in beiden Modellen wurden unterschiedliche Parameter der Erkrankung untersucht. Diese sind für akute Colitis in Tabelle 2 und für chronische Colitis in Tabelle 3 zusammengefasst.

Der Heidelbeerextrakt 25 % zeigt bei der akuten Colitis einen sehr positiven Einfluss auf die Entwicklung der Darmlänge, wogegen die getrockneten Heidelbeeren kaum einen Einfluss aufweisen. Auf den Gewichtsverlauf zeigen die getrockneten Heidelbeeren ebenfalls einen negativen Effekt, verglichen mit dem Heidelbeerextrakt 25 %, der sogar zu einer Gewichtszunahme führt. Durch beide hier verwendeten Vaccinium-Zubereitungen (getrocknete Heidelbeeren und Heidelbeerextrakt 25 %) wird die Sekretion der proinflammatorischen Cytokine IL-17 und TNF reduziert. Die Ergebnisse deuten auf eine positive Beeinflussung des Krankheitsverlaufes der akuten Colitis durch Anwendung von Heidelbeerextrakt 25 %. Die getrockneten Heidelbeeren zeigen sich für dieses Krankheitsbild als ungeeignet. Die Ursache für die bessere Wirkung des Heidelbeer-Extraktes 25 % im Fall der akuten Colitis könnte der hohe Gehalt an Anthocyanen sein, die in der Literatur für ihre anti-inflammatorische und antioxidative Wirkung beschrieben sind.

Überraschend wurde jedoch festgestellt, dass die einzelnen Parameter des Krankheitsverlaufes der chronischen Colitis ganz anders als bei der akuten Colitis durch die getrockneten Heidelbeeren positiv beeinflusst werden (Tabelle 3). Bei der Auswertung des histologischen Scores wurde festgestellt, dass Heidelbeerextrakt 25 % eine leichte Verbesserung bewirkt, was im weiteren bedeutet, dass die Schädigung der Darmschleimhaut etwas weniger ausgeprägt ist, als in der Kontrollgruppe. Getrocknete Heidelbeeren führen jedoch zu einer starken Reduktion dieses Entzündungswertes. Der Effekt der getrockneten Heidelbeeren, liegt dabei in einer Größenordnung wie der einer TNF- Blockierung oder der Gabe von Steroiden. Das bedeutet, dass hier im chronischen DSS-Modell (im Gegensatz zur akuten DSS-Colitis) eine drastisch verminderte Schädigung der Darmwand auftritt, mit weniger Geschwüren und Ulcerationen, mit einer Erhaltung der Darmbarriere und einer geringeren Entzündung. Damit verhalten sich die getrockneten Heidelbeeren überraschender Weise ähnlich wie z.B.

Steroide und TNF- Hemmer, die ebenfalls im akuten Modell nicht wirken, im chronischen Modell jedoch sehr gut und auch in der klinischen Behandlung so etabliert sind.

Heidelbeerextrakt 25 % führt zu einer starken Reduktion des proinflammatorischen Cytokins IL-17 in T-Zellen, die aus den mesenterialen Lymphknoten der Versuchtiere isoliert wurden. Auf TNF haben die Anthocyane aus dem Heidelbeerextrakt 25 % keinen Einfluss. Getrocknete Heidelbeeren führen hingegen zu einer starken Reduktion beider Cytokine.

Bevorzugte erfindungsgemäße, der Ernährung oder dem Genuss dienende Zubereitungen sind:
Süßwaren wie z.B. Lutschbonbons, Kaugummis, Fruchtgummis, Kaubonbons, (Erfrischungs-)Dragees, Komprimate, Hartkaramellen, Schokocremes, Konfekt und Schokolade, Backwaren wie Kuchen, Waffeln und Kekse, Snacks, Instant-Mahlzeiten sowie anderen Instant-Produkten (Getränkepulver und -granulate), Speiseeis, Fruchtzubereitungen (Marmelade, Konfitüre, Fruchtsaucen), Desserts (Puddings, Götterspeise), Milchprodukte (Quark, Joghurts, probiotische Joghurts, Milchdrinks, Molkezubereitungen) sowie Cerealien (Cornflakes, Müsli, Müsliriegel).

Besonders bevorzugte erfindungsgemäße, der Ernährung oder dem Genuss dienende Zubereitungen sind Fruchtgummis, Fruchtzubereitungen (Marmelade, Konfitüre, Fruchtsaucen), Milchprodukte (Quark, Joghurts, probiotische Joghurts, Milchdrinks, Molkezubereitungen) sowie Cerealien (Cornflakes, Müsli, Müsliriegel), wobei wiederum die Milchprodukte Joghurts, probiotische Joghurts und Milchdrinks am meisten bevorzugt sind.

Daneben ist auch die Verwendung in Nahrungsergänzungsmitteln und pharmazeutischen Produkten, wie Lutschtabletten, Hals- oder Hustenbonbons, pharmazeutischen Pulvern, Tabletten oder Granulaten vorteilhaft.

Als weitere Bestandteile für erfindungsgemäße, insbesondere der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Amylose, Amylopektin, Inulin, Xylane, Cellulose), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), ätherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, Kühlwirkstoffe wie z.B. Menthol, Mentholderivate (z.B. L-Menthol, L-Menthyllactat, L-Menthylglutarat, L-Menthylsuccinat) oder Cubebol, synthetische, natürliche oder naturidentische Aromastoffe oder Riechstoffe sowie Geruchskorrigentien.

Erfindungsgemäße, insbesondere der Ernährung oder dem Genuss dienende Zubereitungen können zusätzlich ein oder mehrere Geschmackskorrigenzien enthalten, vorzugsweise gewählt aus der folgenden Liste: Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), weitere Hydroxyflavanone (z.B. Eriodictyol, Homoeriodictyol oder deren Natriumsalze), insbesondere gemäß US 2002/0188019, Hydroxybenzoesäureamide nach DE 10 2004 041 496 (z.B. 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxy-benzyl)amid, 2-Hydroxy-benzoesäure-N-4-(hydroxy-3-methoxybenzyl)amid , 4-Hydroxybenzoesäure-N-(4-hydroxy-3-methoxybenzyl)amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)ethylamid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid (Aduncamid), 4-Hydroxybenzoesäurevanillylamid), bittermaskierende Hydroxydeoxybenzoine gemäß WO 2006/106023 sowie den darauf basierenden Dokumenten (Symrise) (z.B. 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon), Aminosäuren (z.B. gamma-Aminobuttersäure nach WO 2005/096841 zur Verminderung oder Maskierung eines unangenehmen Geschmackseindrucks wie Bitterkeit), Äpfelsäureglycoside nach WO 2006/003107, salzig schmeckende Mischungen nach WO 2007/045566, Diacetyltrimere nach WO 2006/058893, Divanillin, Gemische von Molkeproteinen mit Lecithinen und/oder bittermaskierende Substanzen wie Gingerdione gemäß WO 2007/003527.

Erfindungsgemäße, insbesondere der Ernährung oder dem Genuss dienende Zubereitungen können zusätzlich ein oder mehrere Alkamide enthalten, vorzugsweise ausgewählt aus der Gruppe bestehend aus: 2E,4E-Decadiensäure-N-isobutylamid (Pellitorin), 2E,4Z- Decadiensäure-N-isobutylamid (cis-Pellitorin), 2Z,4Z- Decadiensäure-N-isobutylamid, 2Z,4E- Decadiensäure-N-isobutylamid, 2E,4E-Decadiensäure-N-([2S]-2-methylbutyl)amid, 2E,4E-Decadiensäure-N-([2S]-2-methylbutyl)amid, 2E,4E-Decadiensäure-N-([2R]-2-methylbutylamid), 2E,4Z-Decadiensäure-N-(2-methylbutyl)amid, 2E,4E-Decadiensäure-N-piperid (Achilleamid), 2E,4E-Decadiensäure-N-piperid (Sarmentin), 2E-Decensäure-N-isobutylamid, 3E-Decensäure-N-isobutylamid, 3E-Nonensäure-N-isobutylamid, 2E,6Z,8E-Decatriensäure-N-isobutylamid (Spilanthol), 2E,6Z,8E-Decatriensäure-N-([2S]-2-methylbutyl)amid (Homospilanthol), 2E,6Z,8E-Decatriensäure-N-([2R]-2-methyl-butyl)amid, 2E-Decen-4-insäure-N-isobutylamid, 2Z-Decen-4-insäure-N-isobutylamid, Sanshoole.

Die Erfindung wird anhand der nachfolgenden Beispiele exemplarisch dargelegt. Sofern nicht anders angegeben beziehen sich alle Angaben auf das Gewicht.

### Beispiel F1: Zuckerfreie Hartkaramellen mit erfindungsgemäßen Produkten (getrocknete Heidelbeeren)

| ***Inhaltsstoff*** | A (Gew.-%) | B (Gew.-%) |
|---|---|---|
| Palatinit, Typ M | Ad 100 % | Ad 100 % |
| Wasser | 24,82 % | 24,82 % |
| Pfefferminzaroma | 0,15 % | 0,05 % |
| Orangenaroma | - | 0,10 % |
| Hesperetin | - | 0,01 % |
| Spilanthol | - | 0,01 % |
| Trans-Pellitorin | 0,01 % | - |
| Getrocknete Heidelbeeren, feines Pulver | 10 % | 10 % |

Palatinit wurde mit Wasser gemischt und die Mischung bei 165 °C aufgeschmolzen und anschließend auf 115°C abgekühlt. Aroma und erfindungsgemäß getrocknete Heidelbeeren, sowie trans-Pellitorin im Falle A und Spilanthol und Hesperetin im Falle B, wurden zugegeben und nach dem Durchmischen in Formen gegossen, nach dem Erstarren aus den Formen entfernt und anschließend einzeln verpackt.

### Beispiel F2: Fettarme Joghurts mit erfindungsgemäßen Produkten (getrocknete Heidelbeeren)

| | Zubereitung (Angaben in Gew.-%) | | |
|---|---|---|---|
| ***Inhaltsstoff*** | A | B | C |
| Sucrose | 10 % | 8 % | 6 % |
| Tagatose | - | - | 0,5 % |
| Fructose | - | - | 0,5 % |
| Hesperetin | - | 0,01 % | 0,005 % |
| Phloretin | - | - | 0,005 % |
| Pfirsicharoma | 0,30 % | - | 0,40 % |
| Erdbeer-Rhabarberaroma | - | 0,25 % | - |
| Getrocknete Heidelbeeren | 20 % | 20 % | 20 % |
| Joghurt, 0,1 % Fett | Ad 100 % | Ad 100 % | Ad 100 % |

Die Inhaltsstoffe wurden gemischt und bei 5°C geküh It.

### Beispiel F3: Fettarme Joghurts mit erfindungsgemäßen Produkten (getrocknete Heidelbeeren)

| | Zubereitung (Angaben in Gew.-%) | | |
|---|---|---|---|
| ***Inhaltsstoff*** | A | B | C |
| D-Tagatose | 0,482 % | 0,482 % | 0,482 % |
| Sucralose | 0,003 % | 0,003 % | 0,003 % |
| Aspartam | 0,005 % | 0,005 % | 0,005 % |
| Acesulfam K | 0,01 % | 0,01 % | 0;01 % |
| Hesperetin | - | 0,01 % | 0,005 % |
| Phloretin | - | - | 0,005 % |
| Himbeer-Zitronenaroma | 0,30 % | - | 0,40 % |
| Erdbeer-Rhabarberaroma | - | 0,25 % | - |
| Getrocknete Heidelbeeren | 20 % | 20 % | 20 % |
| Joghurt, 0,1 % Fett | zu 100 % auffüllen | zu 100 % auffüllen | zu 100 % auffüllen |

Die Inhaltsstoffe wurden gemischt und bei 5°C geküh It.

### Beispiel F4: Diätschokolade auf Basis von Fructose mit erfindungsgemäßen Produkten (getrocknete Heidelbeeren)

Es wurde eine für Diabetiker geeignete Schokolade aus folgenden Zutaten hergestellt und in rechteckige Tafeln gegossen:
Kakaomasse, Fructose, Magermilchpulver, Kakaobutter, Inulin, Butterreinfett, Emulgator Sojalecithin, Walnüsse, Speisesalz, Joghurt-Vanille-Aroma (enthaltend Vanillin und 1 Gew.-% Hesperetin, bezogen auf das Gesamtgewicht des Vanille-Aromas) und 5 Gew.-% erfindungsgemäßes Produkt (getrocknete Heidelbeere).

Nährwert (pro 100 g):
Eiweiss 8,8 g, Kohlenhydrate 34 g (davon Fructose 23 g, Lactose 7,5 g, Saccharose 1,4 g), Fett 36 g; Ballaststoffe 18,5 (davon 12,2 g Inulin); Natrium: 0,10 g. Kakao-Anteil mindestens 50 Gew.-%.

### Beispiel F5: Müslimischung mit erfindungsgemäßen Produkten (getrocknete Heidelbeeren)

| Nr. | | A | B | C |
|---|---|---|---|---|
| | | (Gew.-%) | (Gew.-%) | (Gew.-%) |
| 1 | Haferflocken | 12,00 | 12,00 | 12,00 |
| 2 | Knusprige Haferflocken Cluster | 10,00 | 9,00 | 9,00 |
| 3 | Reis Crispies | 12,90 | 10,90 | 10,90 |
| 4 | Cornflakes | 16,50 | 9,50 | 9,50 |
| 5 | Korinthen | 3,50 | 3,50 | 3,50 |
| 6 | Haselnüsse, zerhackt | 2,50 | 2,50 | 2,50 |
| 7 | Glucose Sirup aus Weizen, DE 30 | 9,50 | 9,50 | 9,50 |
| 8 | Saccharose | 19,00 | 19,00 | 19,00 |
| 9 | Wasser | 4,00 | 4,00 | 4,00 |
| 10 | Citronensäurepulver, wasserfrei | 0,10 | 0,10 | 0,10 |
| 11 | Getrocknete Heidelbeeren | 20 | 20 | 20 |

Bestandteile Nr. 1 bis 6 in einer Drehtrommel mischen (Mix 1). Bestandteile Nr. 7 bis 9 erwärmen und Bestandteile Nr. 10 bis 12 zugeben (Mix 2). Mix 2 zu Mix 1 geben und gut mischen. Zuletzt die resultierende Müslimischung auf ein Backblech geben und in einem Ofen bei 130°C für 8 Minuten trocknen.

### Beispiel F6: Fruchtgummis mit erfindungsgemäßen Produkten (getrocknete Heidelbeeren)

| Zutaten: | A (Gew.-%) | B (Gew.-%) |
|---|---|---|
| Wasser | 18,70 | 20,70 |
| Saccharose | 34,50 | 8,20 |
| Glucosesirup, DE 40 | 31,89 | 30,09 |
| Iso Syrup C* Tru Sweet 01750 (Cerestar GmbH) | 1,50 | 2,10 |
| Gelatine 240 Bloom | 8,20 | 9,40 |
| Polydextrose (Litesse®) Ultra, Danisco Cultor GmbH) | - | 24,40 |
| Gelber und roter Farbstoff | 0,01 | 0,01 |
| Citronensäure | 0,20 | 0,10 |
| Orangenaroma | - | 0,10 |
| Getrocknete Heidelbeeren | 5,0 | 4,90 |

### Beispiel F7: Kaubonbon mit erfindungsgemäßen Produkten (getrocknete Heidelbeeren)

Zutatenliste:

| Wasser | | 7,7 % |
|---|---|---|
| Zucker | Raffinade C4 | 32,4 % |
| Glucose Sirup | Dextrose 40 | 37,3 % |
| gehärtetes Pflanzenfett | Schmelzpunkt 32-36 °C | 6,6 % |
| Lecithin | Emulgator (Sojalecithin) | 0,3 % |
| Gelatine | Schweinegelatine | 0,8 % |
| Fondant | Typ - so | 4,9 % |
| Getrocknete Heidelbeeren | | 10 % |

### Beispiel F8: Fruchtiger Müsliriegel mit erfindungsgemäßen Produkten (getrocknete Heidelbeeren)

Zutatenliste:

| | | |
|---|---|---|
| Saccharose | Zucker | 16,0 % |
| Glucose Sirup | | 14,0 % |
| Sorbit P 300 | Feuchthaltemittel | 5,0 % |
| Pflanzenfett | | 5,0 % |
| Wasser | | 3,0 % |
| Haferflocken | | 7,3 % |
| Haferflakes | Hafer Extrudate | 7,0 % |
| Cornflakes | | 4,5 % |
| Rice Crispies | Reis Extrudate | 15,0 % |
| Korinthen | | 3,0 % |
| Getrocknete Heidelbeeren | | 20 % |
| Zitronensäure, Puder | | 0,2 % |

### Beispiel F9: Waffel-Fettfüllung mit erfindunasaemäßen Produkten (getrocknete Heidelbeeren)

Zutatenliste:

| | | |
|---|---|---|
| Pflanzenhartfett | Schmelzpunkt 33 - 35°C | 35,7 % |
| Puderzucker | | 35,0 % |
| Dextrose | Traubenzucker, | 19,0 % |
| | | |
| | wasserfrei, mikrofein | |
| Citronensäure | | 0,3 % |
| Getrocknete Heidelbeeren | | 10 % |

Herstellungshinweise: Das Fett auf Raumtemperatur / ca. 21°C temperieren. Den Puderzucker fein absieben. Alle Zutaten, inklusive Aroma, in einen Hobart-Laborrührer aufschlagen.

### Beispiel F10: Sandkuchen mit erfindunasaemäßen Produkten (getrocknete Heidelbeeren)

Zutatenliste / Basisrezeptur Sandkuchen:

| | | |
|---|---|---|
| Weizenmehl | Typ 405 | 10,90 % |
| Weizenstärke | | 4,90 % |
| Saccharose | Zucker, EG Qualität I | 15,20 % |
| Kochsalz | | 0,14 % |
| Kartoffelmehl | | 7,09 % |
| Eigelbpulver | | 1,84 % |
| Backpulver | | 0,70 % |
| Aufschlagemulgator | Mono-Diglyceride | 1,42 % |
| Wasser | | 10,48 % |
| Reinfett | Schmelzpunkt ca. 34 °C | 10,89 % |
| Eier | | 15,02 % |
| beta-Carotin, 1 %ige Lösung | | 1,42 % |
| Getrocknete Heidelbeeren, ganz oder Pulver | | 20 % |

Herstellungshinweise:
Das Fett temperieren. Alle Trockenstoffe in die Rührschüssel des Hobart-Labormixers geben. Dann Reinfett, Wasser und Eier hinzugeben (Eiergewicht ca. 60 g / Stück). Zuletzt 20 Gew.-% erfindungsgemäßes Produkt (getrocknete Heidelbeeren oder feines Pulver daraus) zugeben und 1 Minute auf Stufe 1 und 2 Minuten auf Stufe 3 aufschlagen. Den Teig in eine Backform geben und 55 Minuten bei 180°C backen.

### Beispiel F11: Mürbeteigkekse (industriequalität) mit erfindungsgemäßen Produkten (getrocknete Heidelbeeren)

Zutatenliste:

| Weizenmehl | Typ 550 | 45,0 % |
|---|---|---|
| Pflanzenweichfett | Schmelzpunkt 24 / 26 °C | 19,0 % |
| Puderzucker | | 18,0 % |
| Salz | | 0,4 % |
| Ammoniumbicarbonat | Triebmittel | 0,4 % |
| Magermilchpulver | | 1,0 % |
| Maltosesirup | DE 60,5 | 1,2 % |
| Wasser | | 5,0 % |
| Getrocknete Heidelbeeren | | 10 % |

Herstellungshinweise:
a) Puderzucker, Maltosesirup, Magermilchpulver und Pflanzenweichfett im Hobart-Laborkneter auf Stufe 1 glattlaufen lassen.
b) Mit einem Teil des Wassers das Ammoniumbicarbonat lösen und das restliche Wasser zu a. geben und kurz mischen.
c) Die restlichen Zutaten mit getrockneten Heidelbeeren aus Beispiel 1 zu Mischung a) geben und zu einem glatten Teig ausarbeiten.
d) Den Teig mit der Ausrollmaschine auf ca. 3 mm Dicke ausrollen, evtl. mit einem Reliefholz ein Muster aufbringen, und in der gewünschten Form ausstechen.

Enddicke des Teiges: ca. 2,6 mm; Ofentemperatur: 200°C, Backzeit: 6 Minuten.

### Beispiel F12: Halsbonbons mit flüssig-viskoser Kernfüllung (centre-filled hard candy)

| | I (Gew.-%) | II (Gew.-%) |
|---|---|---|
| Mischung A (Hülle) (80% der Bonbons) | | |
| Zucker (Saccharose) | 58,12 | 49,37 |
| Glucosesirup (Feststoffgehalt 80%) | 41,51 | 49,37 |
| Pfefferminzöl | 0,10 | 0,15 |
| I-Menthylsuccinat | 0,07 | 0,10 |
| I-Menthol | 0,10 | - |
| Citronenöl | 0,10 | 0,10 |
| Citronensäure | - | 0,91 |
| Total (Hülle): | 100 | 100 |
| Mischung B (Kern) (20% der Bonbons) | | |
| High Fructose Maissirup (Gehalt an festen Zuckern 85%, knapp 15% Wasser) | 83,42 | 83,38 |
| Glycerin | 15,00 | 14,50 |
| Getrocknete Heidelbeeren | 1,00 | 1,50 |
| Lecithin | 0,02 | 0,02 |
| Zimtöl | - | 0,30 |
| Eucalyptusöl | 0,28 | - |
| Trans-Pellitorin | 0,01 | - |
| Vanillylalkohol-n-butylether | - | 0,10 |
| Roter Farbstoff, als 5%ige wässrige Lösung | 0,20 | 0,20 |
| Vanillin | 0,07 | - |
| Total (Kern): | 100 | 100 |

In Anlehnung an die in US 6,432,441 (dort Beispiel 1) sowie die in US 5,458,894 bzw. US 5,002,791 beschriebenen Verfahren wurden Bonbons mit flüssig-viskosem Kern hergestellt. Beide Mischungen A und B wurden separat zu Basen für Hülle (Mischung A) bzw. Kern (Mischung B) verarbeitet. Die mittels Co-Extrusion erhaltenen gefüllten Halsbonbons wirkten bei betroffenen Personen beim Verzehr gegen Husten, Halsschmerzen und Heiserkeit.

### Beispiel TS: Teststudie

### TS1: Akute Colitis (Referenzbeispiel, nicht erfindungsgemäß)

Es wurde der Einfluss von (a) getrockneten Heidelbeeren (erfindungsgemäßes Produkt) und (b) Vacciniumfruchtextrakt 25 % (Vergleichsprodukt) auf die akute Colitis untersucht. Als "Vacciniumfruchtextrakt 25%" wurde ein Extrakt von Heidelbeeren mit einem Gesamtgehalt an Anthocyanen von mindestens 25 Gew. %, bezogen auf das Gesamtgewicht des Extraktes, eingesetzt. (Heidelbeerextrakt 25 %, Kaden Biochemicals GmbH).

Die akute Colitis wurde durch Gabe von Trinkwasser mit einem Anteil von 3 % DSS über sieben Tage induziert; es wurden Balb/c-Mäuse eingesetzt.

Nach Induzierung der akuten Colitis wurden die Balb/c-Mäuse in Gruppen a), b) und c) aufgeteilt und für zwei Wochen gefüttert mit:
a) 20 % getrockneten Heidelbeeren, Rest Normalfutter,
b) 10 % Vacciniumfruchtextrakt 25 %, Rest Normalfutter,
c) Normalfutter.

Im Anschluss daran wurden folgende Parameter ausgewertet: Darmlänge, Gewichtsverlauf, Sekretion der proinflammatorischen Cytokine IL-17 und TNF.

Die Studienergebnisse sind der beigefügten Tabelle 2 zu entnehmen.

Der Vacciniumfruchtextrakt 25 % (Heidelbeerextrakt 25 %, Kaden Biochemicals GmbH)) zeigte bei der akuten Colitis einen sehr positiven Einfluss auf die Entwicklung der Darmlänge, wogegen die getrockneten Heidelbeeren kaum einen Einfluss hatten. Auf den Gewichtsverlauf zeigten die getrockneten Heidelbeeren einen recht deutlich negativen Effekt, verglichen mit dem Vacciniumfruchtextrakt 25 %, der zu einer Gewichtszunahme führte. Der Einsatz jeder der beiden verwendeten Vaccinium-Zubereitungen (getrocknete Heidelbeeren; Heidelbeerextrakt 25 %) reduzierte die Sekretion der proinflammatorischen Cytokine IL-17 und TNF.

Die Ergebnisse deuten insgesamt auf eine positive Beeinflussung des Krankheitsverlaufes der akuten Colitis durch Anwendung von Vacciniumfruchtextrakt 25 % hin. Die getrockneten Heidelbeeren hingegen erwiesen sich als für die Behandlung einer akuten Colitis ungeeignet. Die Ursache für die bessere Wirkung des Heidelbeer-Extraktes 25 % im Falle der akuten Colitis basiert vermutlich auf dem hohen Gehalt an Anthocyanen, die in der Literatur für ihre antiinflammatorische und antioxidative Wirkung beschrieben sind.

### TS2: Chronische Colitis (erfindungsgemäß)

Es wurde der Einfluss von getrockneten Heidelbeeren und Vacciniumfruchtextrakt 25 % auf die chronische Colitis untersucht. Verwendet wurden analog zur Vorgehensweise gemäß TS1 Mäuse der Mauslinie B/C.

Die Mäuse wurden in Gruppen a), b) und c) aufgeteilt und zunächst zwei Wochen mit
a) 20 % getrockneten Heidelbeeren, Rest Normalfutter,
b) 10 % Vacciniumfruchtextrakt 25 %, Rest Normalfutter,
c) Normalfutter
gefüttert.

Anschließend wurde eine chronische Colitis induziert. Während dieser Zeit erhielten die Mäuse weiterhin das entsprechende Futter a), b) oder c).

Zur Induktion der chronischen Colitis wurden insgesamt vier Behandlungszyklen durchgeführt, in denen jeweils während sieben Tagen DSS appliziert wurde (Gabe von Trinkwasser mit einem Anteil von 2,5 % DSS), wobei diese Phasen unterbrochen waren von jeweils 7-tägigen Pausen, in denen normales Trinkwasser verabreicht wurde. Nach den insgesamt vier Behandlungszyklen entwickelte sich in den Mäusen eine chronische Colitis, die ohne weitere DSS-Gabe über mehr als sechs Wochen anhielt. Zwischen der vierten und sechsten Woche nach der letzten DSS-Gabe war die chronische Colitis am stärksten ausgeprägt. Zu diesem Zeitpunkt wurden die Tiere getötet und folgende Parameter ausgewertet: Darmlänge, der histologische Score, Entzündungswerte, das proinflammatorische Cytokin IL-17, das in diesem Zusammenhang besonders relevant und aussagekräftig ist (Okayasu I., Hatakeyama S., Yamada M., Ohkusa T., Inagaki Y., Nakaya R. , A novel method in the induction of reliable experimental acute and chronic ulcerative colitis in mice, Gastroenterology 1990 Mar, 98(3): 694-702; G. Kojouharoff, W. Hans, F. Obermeier, D. N. Männel, T. Andus, J. Schölmerich, V. Gross, W. Falk, Neutralization of tumour necrosis factor (TNF) but not of IL-1 reduces inflammation in chronic dextran sulphate sodiuminduced colitis in mice, Clin Exp Immunol. 1997, 107(2): 353-358). Die Ergebnisse der Studie sind in der beigefügten Tabelle 3 zusammengefasst.

Überraschend wurde festgestellt, dass die einzelnen Parameter des Krankheitsverlaufes der chronischen Colitis durch Behandlung mit getrockneten Heidelbeeren äußerst positiv beeinflusst werden, also ganz anders als dies bei der akuten Colitis (siehe oben TS1) der Fall ist.

Bei der Auswertung des histologischen Scores wurde festgestellt, dass Heidelbeerextrakt 25 % eine (lediglich) leichte Verbesserung bewirkt, was bedeutet, dass die Schädigung der Darmschleimhaut (lediglich) etwas weniger ausgeprägt ist als in der Kontrollgruppe. Getrocknete Heidelbeeren führten jedoch zu einer starken Reduktion dieses Entzündungswertes. Der Effekt der getrockneten Heidelbeeren liegt dabei in etwa in dem Bereich, der auch mittels einer TNF-Blockierung oder durch Gabe von Steroiden erreicht wird. Dies bedeutet, dass im chronischen DSS-Modell (im Gegensatz zum Modell der akuten DSS-Colitis) eine drastisch verminderte Schädigung der Darmwand auftritt, mit weniger Geschwüren und Ulcerationen, bei Erhaltung der Darmbarriere und unter Auftreten nur geringerer Entzündungen. Damit bewirken getrocknete Heidelbeeren überraschenderweise Ähnliches wie z. B. Steroide und TNF-Hemmer, die ebenfalls im akuten DSS-Colitis-Modell nicht wirken, im chronischen Colitis-Modell jedoch sehr gut wirken und auch in der klinischen Behandlung etabliert sind.

Heidelbeerextrakt 25 % führt zu einer starken Reduktion des pro-infammatorischen Cytokins IL-17 in T-Zellen, die aus den mesenterialen Lymphknoten der Versuchstiere isoliert wurden; auf TNF haben die Anthocyane aus dem Heidelbeerextrakt 25% keinen positiven Einfluss. Getrocknete Heidelbeeren führen hingegen zu einer starken Reduktion beider Cytokine.

**Tabelle 1: Anthocyane sowie Anthocyangehalte in Extrakten aus getrockneten sowie frischen Heidelbeeren und in Heidelbeer-Extrakt 25 % (vgl. Fig. 1). Abkürzungen: Dp - Delphinidin, Cy - Cyanidin, Pt - Petunidin, Pn - Peonidin, Mv - Malvidin, Pg-Pelargonidin, gal - galactosid, glc - glucosid, ara - arabinosid.**

| PeakNr¹ | Anthocyan | getrocknete Heidelbeeren | frische Heidelbeeren | Heidelbeer-Extrakt 25 % |
|---|---|---|---|---|
| | | [g/kg TM ± s] | [g/kg TM ± s] | [g/kg] |
| 1 | Dp-3-gal | 0,09 ± 0,01 | 12,95 ± 0,86 | 44 |
| 2 | Dp-pentosehexose | nicht quantifiziert | - | - |
| 3 | Dp-3-glc | 0,45 ± 0,05 | 9,11 ± 0,43 | 58 |
| 4 | Cy-3-gal | 0,04 ± 0,01 | 8,69 ± 0,31 | 54 |
| 5 | Dp-3-ara | 0,18 ± 0,02 | 6,73 ± 0,39 | 41 |
| 6 | Cy-3-glc | 0,34 ± 0,03 | 12,92 ± 0,27 | 104 |
| 7 | Pt-3-gal | 0,07 ± 0,01 | 5,44 ± 0,39 | 21 |
| 8 | Cy-3-ara | 0,11 ± 0,01 | 6,00 ± 0,27 | 31 |
| 9 | Pt-3-glc | 0,45 ± 0,05 | 7,50 ± 0,87 | 31 |
| 10 | Pn-3-gal | nicht quantifiziert | nicht quantifiziert | - |
| 11 | Pt-3-ara | 0,21 ± 0,02 | 5,32 ± 0,42 | 26 |
| 12 | Pn-3-glc | 0,08 ± 0,01 | 7,75 ± 0,29 | 31 |
| 13 | Mv-3-gal | 0,11 ± 0,01 | 6,75 ± 1,22 | 55 |
| 14 | Mv-3-glc | 0,84 ± 0,10 | 6,35 ± 0,36 | 42 |
| 15 | Mv-3-ara | 0,64 ± 0,13 | 5,80 ± 0,59 | - |
| 16 | Pn-pent | nicht quantifiziert | - | - |
| 17 | Mv-pent | nicht quantifiziert | - | - |
| ¹ siehe Fig. 1, 2 | | | | |

**Tabelle 2: Parameter für die Beurteilung des Krankheitsverlaufes bei akuter Colitis (Referenzbeispiel)**

| | Darmlänge [cm] | Gewichtsverlauf [g] | IL-17 [pg/ ml] | TNF [pg/ ml] |
|---|---|---|---|---|
| Kontrolle | 9,8 | +2 | 38 | 143 |
| Getrocknete Heidelbee ren | 8,5 | -16 | 27 | 100 |
| Heidelbeerextrakt 25 % | 12,2 | +7 | 20 | 122 |

**Tabelle 3: Parameter für die Beurteilung des Krankheitsverlaufes bei chronischer Colitis**

| | Darmlänge [cm] | Histologischer Score | IL-17 [pg/ ml] | TNF [pg/ ml] |
|---|---|---|---|---|
| Kontrolle/ 1 | 10 | 3,0 | 290 | 260 |
| getrocknete Heidelbee ren/ 1 | 11 | 1,25 | 175 | 170 |
| Kontrolle/ 2 | 10 | 3,5 | 225 | 210 |
| Heidelbeerextrakt 25 %/ 2 | 13 | 3,0 | 80 | 230 |

## Patentansprüche

1. Produkt auf Basis von getrockneten Vacciniumfrüchten, wobei das Produkt ausgewählt ist aus der Gruppe bestehend aus:
(i) getrockneten Vacciniumfrüchten,
(ii) getrockneten Teilen von Vacciniumfrüchten oder
(iii) Extrakten davon,
zur Anwendung in der therapeutischen Beeinflussung von Zuständen des Darmes, wobei die Beeinflussung keine Behandlung der akuten Colitis ist und der Vorbeugung oder Behandlung chronisch entzündlicher Darmerkrankungen, insbesondere chronischer Colitis (*colitis ulcerosa*) dient.

2. Produkt zur Anwendung nach Anspruch 1, wobei die getrockneten Vacciniumfrüchte ausgewählt sind aus der Gruppe bestehend aus:
• amerikanische Heidelbeere, Kulturheidelbeere *(Vaccinium corymbosum*)*;*
• Preiselbeere *(Vaccinium vitis-idaea*);
• gewöhnliche Moosbeere (*Vaccinium oxycoccos*);
• großfruchtige Moosbeere, Kranbeere (cranberry) *(Vaccinium macrocarpon*);
• kleinfrüchtige Moosbeere (*Vaccinium microcarpum*);
• Alpen Rauschbeere (*Vaccinium gaultheroides*);
• Rauschbeere *(Vaccinium uliginosum);*
• Big Huckleberry *(Vaccinium membranaceum*);
• Red Huckleberry (*Vaccinium parvifolium*);
• Sparkleberry (*Vaccinium arboreu*);
• Ohelo-Beere *(Vaccinium reticulatum*);
• kanadische Blaubeere (*Vaccinium myrtilloides*);
• und deren Mischungen.

3. Produkt zur Anwendung einem der vorhergehenden Ansprüche, wobei die getrockneten Vacciniumfrüchte einen Feststoffgehalt von 90 Gew.-% oder mehr besitzen.

4. Verwendung eines Produktes auf Basis von getrockneten Vacciniumfrüchten, ausgewählt aus der Gruppe, bestehend aus:
(i) getrockneten Vacciniumfrüchten,
(ii) getrockneten Teile einer Vacciniumfrucht oder
(iii) Extrakten davon,
zur Herstellung eines Medikaments zur Anwendung in der therapeutischen Beeinflussung von Zuständen des Darmes, wobei die Beeinflussung keine Behandlung der akuten Colitis ist und der Vorbeugung oder Behandlung chronisch entzündlicher Darmerkrankungen, insbesondere chronischer Colitis (*colitis ulcerosa*) dient.

## Claims

1. Product based on dried Vaccinium fruits, wherein the product is selected from the group consisting of:
(i) dried Vaccinium fruits,
(ii) dried parts of Vaccinium fruits, or
(iii) extracts thereof,
for use in the therapeutic influence of conditions of the intestine, wherein the influence is not the treatment of acute colitis and serves the prevention or the treatment of chronic inflammatory bowel diseases, in particular chronic colitis (*ulcerative colitis*)*.*

2. Product for use according to claim 1, wherein the dried Vaccinium fruits are selected from the group consisting of:
• American highbush blueberry, cultivated blueberry (*Vaccinium corymbosum*)*;*
• cowberry (*Vaccinium vitis-idaea*)*;*
• common cranberry (Vaccinium oxycoccos);
• large cranberry, bearberry (cranberry) (*Vaccinium macrocarpon*);
• small Cranberry (*Vaccinium microcarpum*);
• false berry (*Vaccinium gaultheroides*);
• bog blueberry (*Vaccinium uliginosum*);
• big huckleberry (*Vaccinium membranaceum*);
• red huckleberry (*Vaccinium parvifolium*);
• sparkleberry (*Vaccinium arboreum*);
• ohelo berry (*Vaccinium reticulatum*);
• canadian blueberry (*Vaccinium myrtilloides*);
• and mixtures thereof.

3. Product for use according to one of the preceding claims, wherein the dried Vaccinium fruits have a solids content of 90 wt.-% or more.

4. Use of a product based on dried Vaccinium fruits selected from the group consisting of:
(i) dried Vaccinium fruits,
(ii) dried parts of Vaccinium fruits, or
(iii) extracts thereof,
for the manufacture of a medicament for use in the therapeutic influence of conditions of the intestine, wherein the influence is not the treatment of acute colitis and serves the prevention or the treatment of chronic inflammatory bowel diseases, in particular chronic colitis (*ulcerative colitis*).

## Revendications

1. Produit à base de fruits du genre *Vaccinium* séchés, le produit étant choisi dans le groupe constitué par :
(i) les fruits du genre *Vaccinium* séchés,
(ii) les parties de fruits du genre *Vaccinium* séchées ou
(iii) leurs extraits,
destiné à une utilisation pour influencer thérapeutiquement des états de l'intestin, l'influence n'étant pas un traitement de la colite aigue et servant à la prévention ou au traitement de maladies intestinales inflammatoires chroniques, notamment la colite chronique *(colitis ulcerosa*)*.*

2. Produit destiné à une utilisation selon la revendication 1, dans lequel les fruits du genre *Vaccinium* séchés sont choisis dans le groupe constitué par :
• les myrtilles américaines ou myrtilles cultivées (*Vaccinium corymbosum*);
• les airelles rouges (*Vaccinium vitis-idaea*);
• les airelles canneberges (*Vaccinium oxycoccos*);
• les airelles à gros fruits (« cranberry» en anglais) (*Vaccinium macrocarpon*);
• les airelles à petits fruits (*Vaccinium microcarpum*);
• les myrtilles de loup des Alpes (*Vaccinium gaultheroides*);
• les myrtilles de loup (*Vaccinium uliginosum*);
• les airelles à feuilles membraneuses (« big huckleberries» en anglais) (*Vaccinium membranaceum*) *;*
• les airelles à fruits rouges (« red huckleberries» en anglais) (*Vaccinium parvifolium*) *;*
• les airelles en arbre (« sparkleberries» en anglais) (*Vaccinium arboreum*);
• les baies Ohelo (*Vaccinium reticulatum*);
• les airelles fausses myrtilles (*Vaccinium myrtilloides*);
• et leurs mélanges.

3. Produit destiné à une utilisation selon l'une quelconque des revendications précédentes, dans lequel les fruits du genre *Vaccinium* séchés présentent une teneur en solides de 90 % en poids ou plus.

4. Utilisation d'un produit à base de fruits du genre *Vaccinium* séchés, choisis dans le groupe constitué par :
(i) les fruits du genre *Vaccinium* séchés,
(ii) les parties d'un fruit du genre *Vaccinium* séchées ou
(iii) leurs extraits,
pour la fabrication d'un médicament destiné à une utilisation pour influencer thérapeutiquement des états de l'intestin, l'influence n'étant pas un traitement de la colite aiguë et servant à la prévention ou au traitement de maladies intestinales inflammatoires chroniques, notamment la colite chronique *(colitis ulcerosa*)*.*
